# EUROPÄISCHE PATENTSCHRIFT

(11) **EP 1 199 560 B1**
(45) Veröffentlichungstag und Bekanntmachung des Hinweises auf die Patenterteilung: **09.09.2009**
(21) Anmeldenummer: 01116289.8
(22) Anmeldetag: 05.07.2001
(51) Int. Cl.: G01N 27/327, C12Q 1/00, G01N 33/543, C12Q 1/04

(54) **Elektrochemisches Verfahren zur Bestimmung der Zellzahl und Aktivität von biologischen Systemen**
Electrochemical method for the determination of the cell count and the biological activity
Procédé électrochimique pour la détermination du nombre de cellules et l'activité des systèmes biologiques

(30) Priorität: 07.10.2000 DE 20017268 U
(43) Veröffentlichungstag der Anmeldung: 24.04.2002
(73) Patentinhaber: DECHEMA Gesellschaft für Chemische Technik und Biotechnologie e.V., 60486 Frankfurt (DE)
(72) Erfinder: Sell, Dieter, Dr., 61206 Wöllstadt (DE); Pescheck, Michael, Dipl.-Ing., 63619 Bad Orb (DE)
(74) Vertreter: Meyer-Dulheuer, Karl-Hermann

(56) Entgegenhaltungen:
- EP-A- 0 668 502
- WO-A-00/20626
- DE-U- 29 910 595
- GB-A- 2 173 313
- US-A- 4 528 270
- ABDEL-HAMID ET AL: "Fast amperometric assay for E. coli 0157:H7 using partially immersed immunoelectrodes" ELECTROANALYSIS, Bd. 10, Nr. 11, September 1998 (1998-09), Seiten 758-763, XP000973109

## Beschreibung

Gegenstand der Erfindung ist ein Verfahren zur Bestimmung der Zellzahl und Aktivität von biologischen Systemen, die beispielsweise in der Lebensmittelindustrie zur Überwachung der Qualität von Starterkulturen eingesetzt werden kann.

Eine Qualitätskontrolle der eingesetzten Starterkultur erfolgt sowohl beim Hersteller auch beim Anwender meist in Form traditioneller Methoden der Keimzahlbestimmungen zum Beispiel durch einen Plattentest, der jedoch ein Ergebnis erst mit mehrtägiger Verzögerung liefert.

Im Hinblick auf die Optimierung betrieblicher Produktionsabläufe stellte sich deshalb die Aufgabe, wesentlich schneller und effektiver als derzeit möglich Angaben zur Tauglichkeit mikrobieller Kulturen zu erhalten. Dies gilt sowohl für den Hersteller von Starterkulturen als auch für den Anwender.

Hinzu kommt, dass auch ein erhebliches Interesse an einer schnellen Messmethode zur Bestimmung der Qualität von Lebensmitteln oder Lebensmittelkonzentraten besteht. Die Vermehrung von Mikroorganismen in Lebensmitteln oder Lebensmittelkonzentraten führt in der Regel zu Qualitätseinbußen durch Veränderung von Geruch und Geschmack und den Verlust wertgebender Inhaltsstoffe. Insbesondere für weiterverarbeitende Betriebe ist es deshalb sehr wichtig, eine Aussage über die Keimbelastung der in der Lebensmittelindustrie eingesetzten Grundstoffe zu erhalten, damit noch während des Produktionsprozesses geeignete Maßnahmen ergriffen werden können oder nach dem Erkennen einer Kontamination der Rohware diese erst gar nicht verarbeitet wird.

Es sind bereits zahlreiche mikrobiologische Untersuchungsmethoden bekannt, die in der Lebensmittelindustrie eingesetzt werden können. Eine Kontrolle der Keimkonzentrationen kommerzieller mikrobiologischer Starterkulturen erfolgt in der Regel über Ausplattieren auf geeigneten Nährmedien nach entsprechender Verdünnung des Ausgangspräparates. Anhand der sich nach einigen Tagen auf den Nähragrarplatten entstehenden Kolonien kann die ursprüngliche Keimkonzentration zurückgerechnet werden. Andere Methoden wie das Zählkammerverfahren oder eine photometrische/turbidmetrische Bestimmung führen schneller zu einem Ergebnis, jedoch kann mit diesen Verfahren nicht oder nur sehr beschränkt festgestellt werden, ob die zu untersuchende Probe tatsächlich lebende, aktive Zellen enthält. Auch können kleine Zellzahlen mit diesen Techniken nicht bestimmt werden.

Bei geringen Keimgehalten besteht die Möglichkeit, die Mikroorganismen auf einer Membran abzuscheiden und eine mikroskopische Direktzählmethode, zum Beispiel mittels Epifluoreszenz-Mirkoskopie, durchzuführen. Diese Methode führt zwar zu schnellen Ergebnissen, kann sich aber bislang nicht durchsetzen, da das Färbeverhalten der Mikroorganismen sowie die Auszähltechnik noch mit Schwierigkeiten behaftet sind. Eine Variante dieses Verfahrens besteht aus einem Lasersystem, welches eine Filtrationsmembran nach Inkubation mit einem Fluroeszenzfarbstoff automatisch abrastert und vitale Keime identifiziert.

Ein weiteres Verfahren, welches im Bereich der Lebensmittel-Qualitätskontrolle bereits zum Stand der Technik gehört (DIN 10195 zur Keimzahlbestimmung in Milch) ist das Coulter-Counter-Verfahren. Das Coulter-Prinzip beruht auf einer Widerstandsmessung. Durch eine sich in einem elektrischen Feld befindliche Kapillare fließt ein definierter elektrischer Strom. Dieser Stromfluss verändert sich, wenn ein Partikel, zum Beispiel ein Mikroorganismus, die Kapillare passiert, da die Leitfähigkeit des Partikels sich von der des Elektrolyten unterscheidet. Die Anwendung dieser Methode ist immer dann mit Schwierigkeiten verbunden, wenn sich neben Mikroorganismen weitere Partikel im Flüssigmedium befinden, die zu Falschsignalen führen können.

Weiterhin steht als Schnellmethode das Biolumineszenz-Messverfahren zur Verfügung. Dieses Verfahren hat sich bis jetzt allerdings nicht durchsetzen können. Die unterschiedlichen ATP-Gehalte und -Umsatzraten in Abhängigkeit von den jeweiligen Mikroorganismen sind zu unterschiedlich, so dass produktbezogene Eichkurven bzw. Kennlinien erarbeitet werden müssten. Für Messungen von Keimbelastungen bei Rohwaren oder in abgefüllten Getränken konnten mit dieser Methode keine befriedigenden Resultate erzielt werden; lediglich bei der Reinigung von Tanks und Leitungen in der Getränkeindustrie konnten Erfolge erzielt werden, da Differenzmessungen vor oder nach den Reinigungsschritten eine Bewertung ermöglichten.

Schließlich sind auch bereits immunbiologische Verfahren zur Detektion und zur Bestimmung von Kontaminationskeimen entwickelt worden. Diese Verfahren werden so durchgeführt, dass polyklonale Antikörper gegen verschiedene Stämme von Mikroorganismen in einem ELISA-Test oder nach Bindung an eine Quarzoberfläche für piezoelektrische Messungen eingesetzt werden. Der Nachteil dieser Testmethode ist darin zu sehen, dass nur die Mikroorganismen erfasst werden, gegen die die Antikörper gerichtet sind, während alle übrigen unerkannt bleiben.

Außerdem ist auch schon die Detektion und Identifizierung von Mikroorganismen durch spezifische DNA-Sonden beschrieben worden. Hierbei werden Zeilen, die durch Filtration aus einer Probe abgetrennt wurden, lysiert und deren DNA extrahiert. Die DNA wird mittels der Polymerase-Kettenreaktion (PCR) amplifiziert und schließlich über Hybridisierungen mit bekannten DNA-Fragmenten bestimmten Mikroorganismen zugeordnet. Hierfür sind bereits Chiptechnologien beschrieben, mit deren Hilfe zeitgleich einige hundert Organismen identifiziert werden können.

Als Nachteil dieser technisch sehr anspruchsvollen Methode ist zu sehen, dass eine Unterscheidung zwischen lebenden und toten Zellen nicht vorgenommen werden kann. Weiterhin ist eine Bestimmung der Anzahl von Zellen kaum möglich. Hinzu kommt, dass die Lyse von Mikroorganismen und die Freisetzung der DNA nicht bei allen Spezies in gleicher Weise erfolgreich ist, wodurch es zu Falschaussagen kommen kann. Schließlich stellt die Extraktion und Isolierung von DNA einen arbeitsintensiven Prozessschritt dar, der hohe Anforderungen an die vorhandene Ausstattung und das durchführende Personal stellt, die nicht bei allen Anwendern vorausgesetzt werden können.

Es sind auch bereits elektrochemische Verfahren zur Keimdetektion entwickelt worden. Bei der Qualitätskontrolle von Lebensmitteln, insbesondere bei Milch, aber auch bei Fleisch, Geflügel und Gemüse werden zur Beurteilung von mikrobiellen Kontaminationen Redoxfarbstoffe eingesetzt und die Zeitdauer des Farbumschlages, der durch die mikrobiellen Dehydrogenasen bewirkt wird, zur Bewertung herangezogen. Diese Methoden liefern nur grobe Richtwerte, sind jedoch für erste orientierende Abschätzungen durchaus sinnvoll und praxisrelevant, da sie ein schnelles Ergebnis liefern.

In mehreren Arbeiten wurden Versuche beschrieben, die mikrobielle Reduktion von Redoxfarbstoffen durch analytische Erfassung der reduzierten Form des Farbstoffs zu quantifizieren, um so eine genaue Aussage zur Beurteilung einer mikrobiellen Kontamination machen zu können. Neben optischen Methoden wurden hierbei in der Vergangenheit auch elektrochemische Methoden erprobt. Beispielsweise beschreiben britische Autoren bereits 1989 die Möglichkeit, einen Mix verschiedener Redoxfarbstoffe einzusetzen, um mit elektrochemischem Messaufbau eine Kurzzeitbestimmung von Mikroorganismen in Milch vorzunehmen. Als problematisch stellte sich hierbei heraus, dass die eingesetzten Redoxfarbstoffe teilweise irreversibel an die Oberflächen der verwendeten Elektroden adsorbierten und diese zunehmend inaktivierten.

Darüber hinaus ist aus dem deutschen Gebrauchsmuster 299 10 595.4 ein elektrochemischer Sensor zur Bestimmung der Anzahl und des physiologischen Aktivitätszustandes von Enzymen, Zellen und Organismen, insbesondere Mikroorganismen, bekannt, der eine Anode und eine Kathode sowie deren elektrische Ableitungen enthält, die über einen äußeren Stromkreis mit einer Vorrichtung zur Messung der Spannung oder des Stromflusses verbunden sind. Der Sensor ist dabei als eine Durchflusszelle mit einem kanalförmigen Reaktionsraum oder als eine allseitig geschlossene Zelle ausgestattet, in der die Untersuchungsflüssigkeit in Bewegung gehalten wird.

Es ist bereits ein Verfahren zur Bestimmung der Zellzahl und Aktivität von biologischen Systemen bekannt (US 4,528,270), wobei in einer mit einer Anode, einer Kathode, deren elektrischen Ableitungen und mit Vorrichtungen zur Messung der Spannung und des Stromflusses ausgerüsteten Messzelle ein Redoxmediator in ein Trägergerüst immobilisiert ist. Aus US-A-4,528,270 lässt sich jedoch nicht entnehmen, dass das zu untersuchende biologische System zusammen mit einem Redoxmediator in einem Trägergerüst immobilisiert ist, wobei zur Aufnahme des Trägergerüsts ein als Gegenelektrode ausgebildetes Gefäß dient.

In US 4,528,270 sind lediglich Enzymelektroden zur elektrochemischen Bestimmung von Stoffen beschrieben, die eine Beschichtung aus u. a. Polyacrylamidgel aufweisen.

Es wurde nun gefunden, dass die Zellzahl und die Aktivität von biologischen Systemen schnell und zuverlässig durch eine elektrochemische Messzelle bestimmt werden kann, die mit einer Anode, einer Kathode, deren elektrischen Ableitungen und mit Vorrichtungen zur Messung der Spannung oder des Stromflusses ausgerüstet ist und das zu untersuchende biologische System zusammen mit einem Redoxmediator in einem aus einem Gel bestehenden Trägergerüst immobilisiert ist.

Das zu untersuchende biologische System kann aus Enzymen, Zellen, Bakterien, Pilzen oder Algen bestehen, beispielsweise aus einer in der Lebensmittelindustrie eingesetzten Starterkultur.

Als Redoxmediatoren werden vorzugsweise Chinon-Hydrochinon-Systeme eingesetzt. Bewährt haben sich das 2, 3, 5, 6-Tetramethyl-p-benzochinon (Durochinon), Juglon, das 2-Hydroxi-1,4-naphthochinon, das 1,2-Naphthochinon, das 1,4-Benzochinon, aber auch andere Redoxmediatoren wie das N-Methylphenazoniummethylsulfat (PMS), das Thionin und das Kaliumhexacyano-ferrat III.

Zur Bildung des Trägergerüstes kann jede gelbildende Substanz eingesetzt werden. Besonders bewährt haben sich Exopolysaccharid-Gele wie das Sephadex-Gel®.

Die in der Messzelle verwendeten Elektroden, die vorzugsweise auch noch eine Referenzelektrode umfassen, können aus jedem zur Herstellung von Elektroden verwendbaren Material bestehen. Ganz besonders bewährt haben sich Elektroden aus einem Metall, aus einem mit Platin beschichteten Titan, aus Tantal, aus Graphit oder Glaskohlenstoff. Als Gegenelektrode kann die Tiegelwand der Messzelle verwendet werden, während die Arbeitselektrode stabförmig gestaltet ist. Die Elektroden können jedoch auch jede andere geometrische Ausgestaltung finden und rechteckig, kreisförmig oder halbkreisförmig sein.

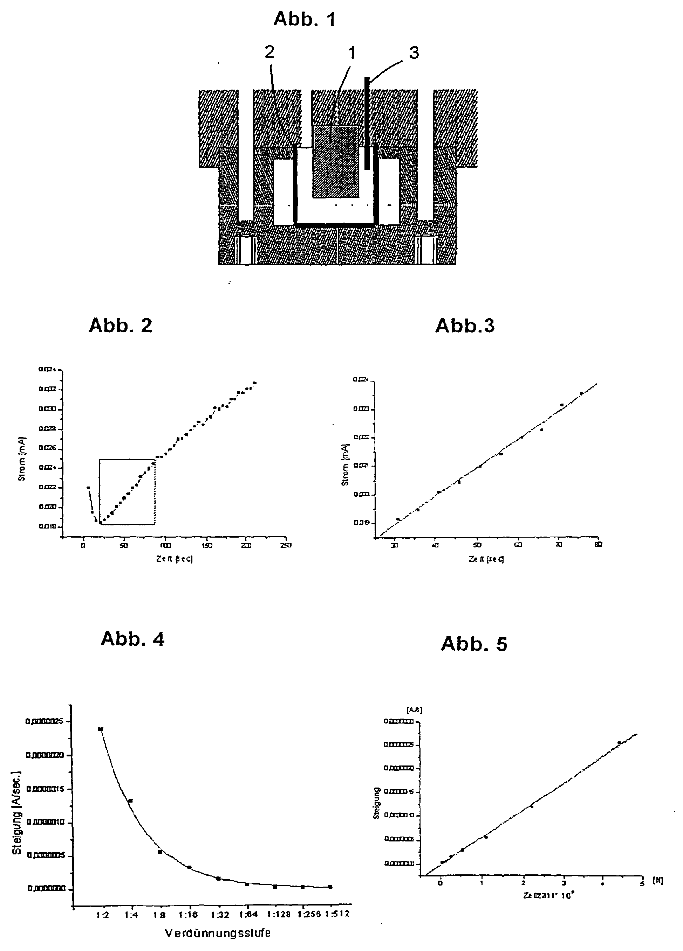

- Abb.1: zeigt einen Querschnitt durch die erfindungsgemäße Messzelle. Die Arbeitselektrode 1 ist dort während der Messung in der Nähe der Gegenelektrode 2 eingetaucht. Als Referenzelektrode 3 (Ag/AgCl, 3 mol KCI) wurde eine Mikroelektrode [MI-401F/Beckford, NH, USA] eingesetzt. Die Messdatenerfassung wurde mittels eines Potentiostaten [Metrhom Pegestat 12/Filderstadt] durchgeführt.

Zur Durchführung der Messung wird als Reaktionsgefäß die zylindrisch geformte Gegenelektrode eingesetzt. In das Reaktionsgefäß werden dann der Redoxmediator, zum Beispiel Durochinon, PMS oder ein anderer Mediator, Glukose und die gelbildende Substanz, zum Beispiel Sephadex [SIGMA G-200-120] vorgelegt, welche zuvor mit einem Mörser fein verrieben wurde. Danach wurden die Arbeits- und die Referenzelektrode in das Reaktionsgefäß eingetaucht. Das biologische System (zum Beispiel eine Hefe, ein Bakterium, ein Enzym oder eine Zelle), welches zuvor in üblicher Verdünnung in einer flüssigen Phase suspendiert wurde, wird dann in das Reaktionsgefäß eingebracht und anschließend vermessen.

Gemessen und ausgewertet wurden die in der erfindungsgemäßen Messzelle festgestellten Stromstärken. Dabei wurde die Steigung der jeweiligen Stromkurve ermittelt und gegen die entsprechende Verdünnungsstufe aufgetragen. Die sich daraus ergebende Kurve ist charakteristisch für den jeweils eingesetzten Organismus und kann somit bei Einhaltung der jeweiligen Prozessparameter als Kalibrierung benutzt werden.
- Abb. 2: zeigt die Aufnahme der Stromkurve einer Kultur mit bestimmter Verdünnungsstufe.
- Abb. 3: zeigt eine lineare Ausgleichsgerade des linearen Bereichs der aufgenommenen Stromkurve zur Ermittlung der Steigung.

Für die Bestimmung der Zellzahl einer neuen Starterkultur des gleichen Stammes ist es nötig, eine Stromkurve der neuen Kultur aufzunehmen. Mit dem Wert der Steigung aus der Stromkurve ist dann in die entsprechende Kalibrierkurve zu gehen. Die ermittelte Steigung ergibt dann entweder eine Verdünnung, in welcher man auch die Zellzahl der Probe errechnen kann (Abb. 4) oder man fertigt eine Kurve an, bei der die Zellzahl direkt ablesbar ist (Abb. 5).

Die Beziehung zwischen Zellzahl und Steigung wurde nach der Erstellung der Kalibrierkurve mittels Kolonie-Bildenden-Einheiten (KBE) hergestellt.

Die Messzelle kann nicht nur für Verfahren zur Zellzahlbestimmung von Starterkurven angewendet werden, sondern dient vielmehr in allen Bereichen, in denen es auf Zellzahlbestimmungen und Aktivitätsbestimmungen von biologischen Systemen ankommt. Besonders geeignet ist die Messzelle zur Zellzahlbestimmung bei Bakterien, Hefen und anderen Pilzen, tierischen Zellen und Mycoplasmen. Sie kann außerdem zur Aktivitätsbestimmung von Enzymen und von gesamten Stoffwechselprozessen eingesetzt werden.

Die besonderen Vorteile des erfindungsgemäßen Verfahrens bestehen darin, dass es in wenigen Sekunden zu einem Ergebnis führt und dass auch Mediatoren in Trägersubstanzen immobilisert werden können, welche entgegengesetzte Polarität zum Lösungsmittel aufweisen. Außerdem wird durch die Immobilisierung eine verbesserte Verteilung im Reaktionsraum auch ohne den Einsatz von Rühreinrichtungen erreicht, welche sonst zur Verteilung der Substanzen erforderlich sind. Vorteilhaft ist außerdem, dass keine Spülung mit einem inerten Gas wie Stickstoff erforderlich ist, um den erneuten Sauerstofftransport in die Messlösung zu verhindern.

## Patentansprüche

1. Verfahren zur Zellzahlbestimmung und/oder Aktivitätsbestimmung von biologischen Systemen, bestehend aus Enzymen, tierischen Zellen, Bakterien, Pilzen oder Algen, in einer elektrochemischen Messzelle, welche mit einer Arbeitselektrode (1), einer zylindrisch geformten Gegenelektrode (2) und einer Referenzelektrode (3) mit Vorrichtungen zur Messung der Spannung und des Stromflusses ausgerüstet ist, **dadurch gekennzeichnet, dass** das Verfahren folgende Verfahrensschritte umfasst:
- Vorlegen eines Redoxmediators und einer gelbildenden Substanz in die als Reaktionsgefäß eingesetzte Gegenelektrode (2),
- anschließendes Eintauchen der Arbeitselektrode (1) und der Referenzelektrode (3) in das Reaktionsgefäß,
- anschließende Einbringung des zuvor in einer flüssigen Phase suspendierten biologischen Systems in das Reaktionsgefäß, sodass das biologische System zusammen mit dem Redoxmediator in der gelbildenden Substanz immobilisiert ist und
- Messung des Stromflusses.

2. Verfahren nach Anspruch 1, **dadurch gekennzeichnet, dass** als gelbildende Substanz Exopolysaccharid-Gele verwendet werden.

3. Verfahren nach den Ansprüchen 1 und 2, **dadurch gekennzeichnet, dass** als Redoxmediatoren Chinon-Hydrochinon-Systeme, N-Methylphenazoniummethylsulfat (PMS), Thionin oder Kaliumhexacyano-ferrat III eingesetzt werden.

4. Verfahren nach Anspruch 3, **dadurch gekennzeichnet, dass** die als Redoxmediatoren verwendeten Chinon-Hydrochinon-Systeme 2, 3, 5, 6-Tetramethyl-p-benzochinon (Durochinon), Juglon, 2-Hydroxi-1,4-naphthochinon, 1,2-Naphthochinon oder 1,4-Benzochinon sind.

## Claims

1. Method for determining cell count and/or determining activity of biological systems, consisting of enzymes, animal cells, bacteria, fungi or algae, in an electrochemical measurement cell, which is equipped with a working electrode (1), a cylindrically-shaped counter electrode (2) and a reference electrode (3) with devices for measuring the voltage and the current flow, **characterized in that** the method comprises the following method steps:
- placement of a redox mediator and of a gel-forming substance into the counter electrode (2), used as reaction vessel,
- subsequent immersion of the working electrode (1) and of the reference electrode (3) into the reaction vessel,
- subsequent introduction into the reaction vessel of the biological system previously suspended in a liquid phase, so that the biological system together with the redox mediator is immobilised in the gel-forming substance and
- measurement of the current flow.

2. Method according to Claim 1, **characterized in that** exopolysaccharide gels are used as gel-forming substance.

3. Method according to Claims 1 and 2, **characterized in that** chinone-hydrochinone systems, N-methyl phenazonium methyl sulphate (PMS), thionine or potassium hexacyanoferrate III are used as redox mediators.

4. Method according to Claim 3, **characterized in that** the chinone-hydrochinone systems which are used as redox mediators are 2,3,5,6-tetramethyl-p-benzochinone (durochinone), juglone, 2-hydroxy-1,4-naphthochinone, 1,2-naphthochinone or 1,4-benzochinone.

## Revendications

1. Procédé de détermination du nombre de cellules et/ou de détermination de l'activité de systèmes biologiques composés d'enzymes, de cellules animales, de bactéries, de champignons ou d'algues, au sein d'une cellule de mesure électrochimique équipée d'une électrode de travail (1), d'une contre-électrode de forme cylindrique (2) et d'une électrode de référence (3) pourvue de dispositifs destinés à mesurer la tension et le courant électrique, **caractérisé en ce que** ledit procédé comprend les étapes suivantes :
- mise en place d'un médiateur d'oxydo-réduction et d'une substance gélifiante dans la contre-électrode (2) employée comme récipient de réaction,
- ensuite, immersion de l'électrode de travail (1) et de l'électrode de référence (3) dans le récipient de réaction,
- ensuite, introduction du système biologique, préalablement mis en suspension dans une phase liquide, dans le récipient de réaction, de façon à ce que ledit système biologique soit immobilisé ensemble avec le médiateur d'oxydo-réduction au sein de la substance gélifiante, et
- mesure du courant électrique.

2. Procédé selon la revendication 1, **caractérisé en ce que** l'on utilise, en tant que substance gélifiante, des gels d'exopolysaccharide.

3. Procédé selon les revendications 1 et 2, **caractérisé en ce que** l'on utilise, en tant que médiateurs d'oxydo-réduction, des systèmes quinone-hydroquinone, du méthylsulfate de N-méthylphénazonium (PMS), de la thionine ou du ferricyanure de potassium.

4. Procédé selon la revendication 3, **caractérisé en ce que** lesdits systèmes quinone-hydroquinone utilisés comme médiateurs d'oxydo-réduction sont de la 2,3,4,6-tétraméthyl-p-benzoquinone (duroquinone), du juglon, de la 2-hydroxy-1,4-naphtoquinone, de la 1,2-naphtoquinone ou de la 1,4-benzoquinone.
